# EUROPÄISCHE PATENTSCHRIFT

(11) **EP 1 084 096 B1**
(45) Veröffentlichungstag und Bekanntmachung des Hinweises auf die Patenterteilung: **01.10.2003**
(21) Anmeldenummer: 99924923.8
(22) Anmeldetag: 10.05.1999
(51) Int. Cl.: C07C 49/297, C07C 45/48

(54) **VERFAHREN ZUR HERSTELLUNG VON CYCLOPENTANON**
METHOD FOR PRODUCING CYCLOPENTANONE
PROCEDE DE PRODUCTION DE CYCLOPENTANONE

(30) Priorität: 28.05.1998 DE 19823835
(43) Veröffentlichungstag der Anmeldung: 21.03.2001
(73) Patentinhaber: BASF AKTIENGESELLSCHAFT, 67056 Ludwigshafen (DE)
(72) Erfinder: LIANG, Shelue, D-67071 Ludwigshafen (DE); FISCHER, Rolf, D-69121 Heidelberg (DE); STEIN, Frank, D-67098 Bad Dürkheim (DE); WULFF-DÖRING, Joachim, D-67227 Frankenthal (DE)
(86) Internationale Anmeldenummer: EP9903194
(87) Internationale Veröffentlichungsnummer: WO99061402

(56) Entgegenhaltungen:
- EP-A- 0 251 111
- DE-A- 3 638 005

## Beschreibung

Die Erfindung betrifft ein verbessertes Verfahren zur Herstellung von Cyclopentanon durch Umsetzung von Adipinsäureestern an oxidischen Festbettkatalysatoren in der Gasphase.

Es ist aus U.S. 2 863 923 bekannt, 2,5-Dialkylcyclopentanone durch Erhitzen von 2,5-Dialkyladipinsäurediestern in der Gasphase in Gegenwart von Metalloxiden, deren Metallkomponente Atomgewichte zwischen 7 und 137 besitzt, herzustellen. Als wirkungsvollste Katalysatoren werden die Oxide des Natriums, Kaliums, Lithiums, Calciums, Bariums, Strontiums, Cadmiums, Zinks, Mangans, Kupfers, Eisens, Nickels und Cobalts genannt. Besonders bevorzugt sind die Oxide des Mangans, Cadmiums und Natriums. Geeignete Katalysatorträger sind Al₂O₃, SiO₂ und Aktivkohle. Die Umsetzung wird bei 350 bis 600°C, bevorzugt 400 bis 475°C, durchgeführt. Gearbeitet wird ohne Zusatz von Wasser und ohne Trägergas. In den Patentbeispielen wird 2,5-Dimethyladipinsäuredimethylester bei 435 bis 465°C an einem MnO₂/Al₂O₃-Katalysator umgesetzt. In weiteren Beispielen werden CdO/Al₂O₃, Na₂O/Al₂O₃ und ZnO/Al₂O₃ (keine Angaben zur Katalysatorzusammensetzung) im Temperaturbereich von 375 bis 502°C verwendet. Angaben zu den 2,5-Dimethylcyclopentanonausbeuten werden nicht gemacht.

Aus EP 251 111 A2 ist bekannt, aliphatische Dicarbonsäureester mit sechs bis acht Kohlenstoffatomen in der Kette unter Zusatz von Wasser und Verwendung von Trägergasen zu entsprechenden Cycloalkanonen umzusetzen.

Als Katalysatoren verwendet man feste oxidische Katalysatoren. Das sind z.B. Oxide von Elementen der I. bis V. Hauptgruppe, der I. bis VIII. Nebengruppe des periodischen Systems der Elemente oder Oxide der seltenen Erdmetalle oder Gemische der genannten Oxide. So sind beispielsweise Erdalkalioxide, wie Magnesiumoxid, Calciumoxid, Bariumoxid, weiterhin Bortrioxid, Aluminiumoxid, Siliciumdioxid, z.B. in Form von Kieselgel, Kieselgur, oder Quarz, ferner Zinndioxid, Bismutoxid, Kupferoxid, Zinkoxid, Lanthanoxid, Titandioxid, Zirkondioxid, Vanadiumoxide, Chromoxide, Molybdänoxide, Wolframoxide, Manganoxide, Eisenoxide, Ceroxide, Neodymoxide oder Gemische derartiger Oxide geeignet. Die Katalysatoren können noch durch Aufbringen von Zusätzen, wie Säuren (z.B. Phosphorsäure) oder Basen (z.B. Natriumhydroxid) modifiziert werden. Bevorzugt sind Magnesiumoxid, Bortrioxid, Aluminiumoxid, Siliciumdioxid, Zinkoxid, Titandioxid, oder deren Gemische, von denen Aluminiumoxidkatalysatoren ganz besonders geeignet sind.

Die höchsten Cyclopentanonselektivitäten werden in den Ausführungsbeispielen 2.1 und 2.7 erreicht. In Beispiel 2.1 wird Adipinsäuredimethylester bei 340°C an γ-Aluminiumoxid im Festbett mit 88%iger Selektivität (Ausbeute 74 %), in Beispiel 2.7 bei 400°C an Li₂O (13,5 %)/MgO mit 91%iger Selekttivität (Ausbeute 62 %) zu Cyclopentanon umgesetzt.

Aus DE-A 36 37 787 ist bekannt, Korksäurediester bei 300 bis 600°C, bevorzugt 400 bis 500°C, unter Zusatz von Wasser oder Alkoholen in der Gasphase an oxidischen Katalysatoren zu Cycloheptanon umzusetzen. Als Katalysatoren werden 5 bis 35 %, insbesondere 8 bis 20 %, Zinkoxid oder Ceroxid auf Aluminiumoxid verwendet. Die höchste Cycloheptanonselektivität wird in Beispiel 5 erreicht. Korksäuredimethylester wird bei 440°C an 12 % ZnO auf Al₂O₃ unter Zusatz von Methanol mit 76 %iger Selektivität (Ausbeute 49 %) zu Cycloheptanon umgesetzt.

Es bestand die Aufgabe, das Verfahren zur Herstellung von Cyclopentanon aus Adipinsäureester, insbesondere im Hinblick auf die Cyclopentanonselektivität durch das Auffinden noch besserer Katalysatoren weiter zu verbessern. Mit einer hohen Cyclopentanonselektivität sollte ein möglichst hohe Cyclopentanon-Ausbeute verbunden sein, um im Verfahren möglichst wenig Adipinsäureester zurückführen zu müssen. Weiterhin sollten die Katalysatoren eine hohe Standzeit aufweisen.

Diese Aufgabe wird gelöst mit einem Verfahren zur Herstellung von Cyclopentanon durch Umsetzung von Adipinsäureestern mit einem Gehalt des Edukts an Adipinsäureester von mehr als 95 %, vorzugsweise über 97 Gew.-% und insbesondere 98,5 bis 100 Gew.-%, der Formel

R₁OOC-(CH₂)₄-COOR₂ I,

in der R₁ und R₂ für Alkylreste mit 1 bis 12 Kohlenstoffatomen, Cycloalkylreste mit 5 oder 6 Kohlenstoffatomen, Aralkyl- oder Arylreste stehen und R₂ zusätzlich Wasserstoff bedeuten kann, in Gegenwart von oxidischen Katalysatoren, wobei man die Umsetzung in der Gasphase in Gegenwart von Wasser, einem Trägergas und
a) 0,01 bis 10 Gew.-% mindestens eines Metalloxids, ausgewählt aus der ersten und zweiten Hauptgruppe des Periodensystems oder der Gruppe der Seltenen Erdmetalle auf Titandioxid oder Zirkondioxid als Katalysatorträger oder
b) 0,01 bis 50 Gew.-% mindestens eines Metalloxids, ausgewählt aus der zweiten Hauptgruppe des Periodensystems auf Zinkoxid als Katalysatorträger durchführt.

Die Umsetzung kann nicht nur in der Gasphase sondern auch weniger vorteilhaft in der flüssigen Phase, gegebenenfalls auch unter Mitverwendung von Verdünnungsmitteln, durchgeführt werden. Als Verdünnungsmittel sind z.B. Lösungsmittel geeignet, die unter den Reaktionsbedingungen vollständig oder weitgehend inert sind, z.B. Ether wie Dioxan oder Tetrahydrofuran.

Die als Ausgangsstoffe benötigten Ester der Formel I sind aliphatische, cycloaliphatische oder aromatische Mono- oder Diester der Adipinsäure. Beispielhaft seien folgende Reste R₁ und R₂ genannt: Methyl-, Ethyl-, Propyl, Isopropyl, Butyl-, Isobutyl, ter.-Butyl-, Hexyl-, Nonyl-, Dodecyl-, Cyclopentyl-, Cyclohexyl-, Phenyl- oder Benzylreste. Besonders bevorzugt ist der Methylrest.

Beispielsweise können folgende Ester als Ausgangsstoffe verwendet werden: Adipinsäuredimethylester, Adipinsäuremonomethylester, Adipinsäurediethylester, Adipinsäuredibutylester, Adipinsäuredicyclohexylester, Adipinsäuredibenzylester, Adipinsäuremonoethylester.

Nach einer besonderen Ausführungsform werden Adipinsäurediester verwendet, die noch geringe Mengen 6-Hydroxycapronsäureester enthalten können, wie sie z.B. nach den in DE-A 19 607 954 beschriebenen Verfahren erhalten werden, wobei geringe Mengen noch weiterer Verbindungen wie Caprolacton, 6-Alkoxycapronsäureester, Glutarsäurediester, 5-Hydroxyvaleriansäureester, 2-Oxocapronsäureester, 1,2-Cylohexandiole, Valerolacton, ungesättigte Adipinsäurediester wie z.B. Dihydromuconsäurediester, 3-Hydroxypentansäureester, 4-Oxopentansäureester und 5-Oxohexansäureester vorliegen können. Diese Verbindungen beeinträchtigen im allgemeinen überraschenderweise weder die erfindungsgemäße Reaktion noch führen sie nach destillativer Aufreinigung zu Einbußen bei der Produktqualität.

In der Regel liegt dabei der Gehalt aus der Summe der Verunreinigungen bei 0,01 bis 4,5 Gew.-%, insbesondere 0,1 bis 3 Gew.-% (bezogen auf Adipinsäurediester).

Es ist zwar möglich, die erfindungsgemäße Umsetzung ohne Zusatz von Wasser durchzuführen, doch wird durch die Zugabe von Wasser eine bemerkenswerte Erhöhung von Selektivität und Standzeit erreicht. Das Molverhältnis von Ester (I) zu Wasser beträgt hierbei vorteilhaft 1:0,05 bis 1:30, insbesondere 1:0,1 bis 1:15.

Es kann weiterhin vorteilhaft sein, zusätzliche Alkohole wie z.B. Methanol, Ethanol, n-Butanol, i-Propanol, n-Propanol zuzusetzen. Dabei wählt man vorteilhaft Alkohole, die den Estern (I) zugrunde liegen. Die Molverhältnisse von Ester (I) zu Alkohol entsprechen den für den Zusatz von Wasser genannten Verhältnissen.

Als Trägergase verwendet man unter den Reaktionsbedingungen inerte Gase wie z.B. Stickstoff, Argon oder Kohlendioxid. Das Molverhältnis von Ester der Formel (I) zu Inertgas beträgt 1:1 bis 1:100, insbesondere 1:10 bis 1:50, bevorzugt 1:20 bis 1:40.

Als Katalysatoren (a) verwendet man Metalloxide der ersten und zweiten Hauptgruppe des Periodensystems oder der Gruppe der Seltenen Erdmetalle auf Titandioxid oder Zirkondioxid als Katalysatorträger.

Beispiele für derartige Metalloxide sind Natriumoxid, Lithiumoxid, Kaliumoxid, Calciumoxid, Magnesiumoxid, Lathanoxid, Ceroxid, Praseodymoxid, Neodymoxid. Besonders bevorzugt sind Natriumoxid, Kaliumoxid und Lanthanoxid.

Die Menge an Metalloxid auf dem Katalysatorträger beträgt 0,01 bis 10 Gew.-%, bevorzugt 0,1 bis 5 gew.-%, besonders bevorzugt 0,1 bis 3 Gew.-% Metalloxid.

Das Titanoxid besitzt eine Oberfläche von 20 bis 200m²/g und besteht überwiegend aus Anatas. Die Herstellung derartiger Katalysatoren ist in EP-A 352 674 beschrieben.

Als Katalysator (b) verwendet man Metalloxide der zweiten Hauptgruppe des Periodensystems auf Zinkoxid als Katalysatorträger.

Beispiele für derartige Metalloxide sind Magnesiumoxid, Calciumoxid, Strontiumoxid und Bariumoxid. Besonders bevorzugt sind Magnesiumoxid und Calciumoxid, Die Menge an Metalloxid auf dem Katalysatorträger beträgt 1 bis 50 Gew.-%.

Die Umsetzung in Gegenwart der Katalysatoren (a) findet bei Temperaturen von 220 bis 350°C, bevorzugt 230 bis 330°C, besonders bevorzugt 250 bis 310°C statt, die der Katalysatoren (b) bei 300 bis 450°C, bevorzugt 330 bis 420°C, besonders bevorzugt bei 350 bis 400°C.

Im allgemeinen wird die Reaktion unter Atmosphärendruck durchgeführt. Es ist jedoch auch möglich, schwach verminderten oder schwach erhöhten Druck, z.B. bis zu 20 bar, anzuwenden. Die Katalysatorbelastung liegt im allgemeinen bei 0,01 bis 40, vorzugsweise 0,1 bis 20 g Ester (I) je Gramm Katalysator und Stunde.

Es ist im Prinzip auch möglich, die Reaktion weniger vorteilhaft in der Flüssigphase mit Festbettkatalysatoren oder suspendierten Katalysatoren durchzuführen. Bevorzugt ist jedoch die erfindungsgemäße Durchführung in der Gasphase. Dabei wird das Arbeiten mit Festbettkatalysatoren vorgezogen, da das Arbeiten mit Wirbelbettkatalysatoren technisch aufwendiger und mit Katalysatorverlusten verbunden ist.

Die bevorzugte Umsetzung in der Gasphase wird beispielsweise so durchgeführt, daß man ein Gemisch aus Ester (I) und Wasser in einen Verdampfer und von dort mit einem Trägergas bei der gewünschten Temperatur über den Festbettkatalysator leitet. Das Reaktionsgemisch wird kondensiert und zur Gewinnung von Cyclopentanon fraktionierend destilliert. Nicht umgesetzter Ester (I) wird abgetrennt und in die Cyclopentanon-Synthesestufe zurückführt.

Mit den erfindungsgemäß zu verwendenden Festbettkatalysatoren werden höhere Cyclopentanonausbeuten bei gleichzeitig hohen Cyclopentanonselektivitäten erreicht als in EP-A 251 111 beschrieben.

### Beispiele

Die Prozentangaben zur Charakterisierung der Katalysatoren bedeuten Gewichtsprozente.

### a) Herstellung der Katalysatoren

- ZrO₂:: Firma Norton (SN 951 6321), als solches direkt eingesetzt.
- La₂O₃(3% La)/ZrO₂:: ZrO₂ (Norton SN 951 6321) wurde mit La(NO₃)₃-Lösung getränkt, vier Stunden bei 120°C getrocknet und sechs Stunden bei 400°C calciniert.
- 44 % CaO/56 % ZnO:: Fa. BASF (H 5-11)

### b) Versuchsdurchführung für die Beispiele in Tabelle 1

In einem elektrisch beheizten Gasphasenreaktor wurden jeweils 100 ml Katalysator mit 30 ml Quarzringen als Verdampferstrecke überschichtet. Unter den angegebenen Bedingungen wurden pro Stunde 10 bis 15 g des in der Tabelle genannten Adipinsäureesters zusammen mit Wasser und Stickstoff von oben nach untern über den Katalysator geleitet. Die Reaktionsausträge wurden in einer Vorlage unter Kühlung mit Trockeneis/Aceton kondensiert. Im Reaktionsaustrag, der während eines Zeitraumes von sieben Stunden gesammelt wurde, ergaben sich die laut gaschromatographischer Analyse gefundenen Cyclopentanon-Ausbeuten und- Selektivitäten (bezogen auf eingesetzten Adipinsäuredimethylester).

| Cyclopentanon aus Adipinsäuredimethylester (ADSME) | | | | | | | |
|---|---|---|---|---|---|---|---|
| Beispiel | Ausgangsstoff I | Katalysator | Katalysator Belastung [kg I/ₗᵢₜₑᵣ Kat x h] | Molverhältnis I: H₂O:N₂ | Temperatur [°C] | Cyclopentanon-Ausbeute [% ] | Cyclopentanon-Selektivität [%] |
| 1 | Adipinsäuredimethylester (Reinheit 98,7%) | H 5-11 | 0,1 | 1:6:10 | 370 | 90,0 | 91,7 |
| 2 | | La₂O₃ (3%La)/ZrO₂ | 0,1 | 1:10:10 | 300 | 82,3 | 84,0 |
| 3 (Vergleich) | | ZrO₂ | 0,1 | 1:5:10 | 350 | 6,5 | 6,6 |
| 4 | | K₂O (2%)/TiO₂ | 0,15 | 1:10:25 | 270 | 77,3 | 93,5 |
| 5 (Vergleich) | | TiO₂ | 0,10 | 1:6:10 | 250 | 56,9 | 73,1 |

### Beispiel 6

In einem elektrisch beheizten Gasphasenreaktor wurden 50 ml Katalysator K₂O/TiO₂ (2 Gew.-% K₂O, Fa. BASF) mit 20 ml Quarzringen als Verdampferstrecke überschichtet. Bei 270°C und Normaldruck wurden pro Stunde 7,5 g Adipinsäuredimethylester und 7,5 g Wasser separat zudosiert und 25 NL Stickstoff zugeleitet. Die Reaktionsausträge wurden in einer Vorlage unter Kühlung mit Trockeneis/Aceton kondensiert. Nach 408 Stunden wurden insgesamt 4774 g Austräge gesammelt. GC-Analytik unter Verwendung eines inneren Standards ergab 12,2 Gew.-% Adipinsäuredimethylester und 24,0 Gew.-% Cyclopentanon. Der Adipinsäuredimethylester-Umsatz betrug hierbei 81 mol-%, die Cyclopentanon-Ausbeute 77,6 mol-% und die Cyclopentanon-Selektivität 95,8 mol-%.

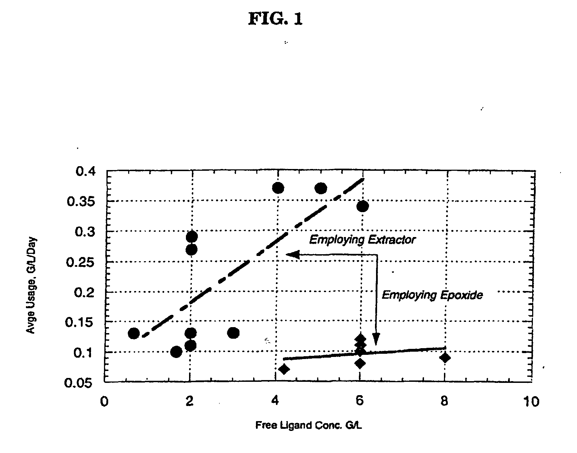

## Patentansprüche

1. Verfahren zur Herstellung von Cyclopentanon durch Umsetzung von Adipinsäureestern der Formel
R₁OOC-(CH₂)₄-COOR₂ I
in der R₁ und R₂ für Alkylreste mit 1 bis 12 Kohlenstoffatomen, Cycloalkylreste mit 5 oder 6 Kohlenstoffatomen, Aralkyl- oder Arylreste stehen und R₂ zusätzlich Wasserstoff bedeuten kann, in Gegenwart von oxidischen Katalysatoren, **dadurch gekennzeichnet, daß** man Adipinsäureester der Formel I mit weniger als 5 Gew.-% Nebenprodukten, die nicht Adipinsäureester sind, in der Gasphase in Gegenwart von Wasser, einem Trägergas und
a) 0,01 bis 10 Gew.-% mindestens eines Metalloxids, ausgewählt aus der ersten oder zweiten Hauptgruppe des Periodensystems oder der Gruppe der seltenen Erdmetalle auf Titandioxid oder Zirkondioxid als Katalysatorträger oder
b) 0,01 bis 50 Gew.-% mindestens eines Metalloxids, ausgewählt aus der zweiten Hauptgruppe des Periodensystems auf Zinkoxid als Katalysatorträger umsetzt.

2. Verfahren nach Anspruch 1, **dadurch gekennzeichnet, daß** man als Edukt Adipinsäureester der Formel I verwendet, die insgesamt 0,01 bis 4,5 Gew.-% Hydroxycapronsäureester und Caprolacton enthalten.

3. Verfahren nach Anspruch 1, **dadurch gekennzeichnet, daß** man als Edukt Adipinsäureester der Formel I verwendet, die insgesamt 0,1 bis 3 Gew.-% Hydroxycapronsäureester und Caprolacton enthalten.

4. Verfahren nach Anspruch 1, **dadurch gekennzeichnet, daß** man die Umsetzung in Gegenwart von Wasser bei einem Molverhältnis Adipinsäureester zu Wasser von 1:0,05 bis 1:30 durchführt.

5. Verfahren nach Anspruch 1, **dadurch gekennzeichnet, daß** man einen Katalysator, ausgewählt aus der Gruppe bestehend aus Natriumoxid, Kaliumoxid oder Lanthanoxid auf den Trägern Titandioxid, oder Zirkondioxid und Magnesiumoxid oder Calciumoxid auf Zinkoxid als Träger verwendet.

6. Verfahren nach Anspruch 1, **dadurch gekennzeichnet, daß** man die Umsetzung in Gegenwart der Katalysatoren (a) bei Temperaturen von 220 bis 350°C durchführt.

7. Verfahren nach Anspruch 1, **dadurch gekennzeichnet, daß** man die Umsetzung in Gegenwart der Katalysatoren (b) bei Temperaturen von 300 bis 450°C durchführt.

## Claims

1. A process for preparing cyclopentanone by reacting adipic esters of the formula
R₁OOC-(CH₂)₄-COOR₂ I
where R₁ and R₂ are each alkyl having from 1 to 12 carbon atoms, cycloalkyl having 5 or 6 carbon atoms, aralkyl or aryl and R₂ may additionally be hydrogen, in the presence of oxidic catalysts, which comprises reacting adipic esters of the formula I having less than 5% by weight of by-products other than adipic esters in the gas phase in the presence of water, a carrier gas and
a) from 0.01 to 10% by weight of at least one metal oxide selected from the first or second main group of the periodic table or from the group of the rare earth metals on titanium dioxide or zirconium dioxide as catalyst support, or
b) from 0.01 to 50% by weight of at least one metal oxide selected from the second main group of the periodic table on zink oxide as catalyst support.

2. A process as claimed in claim 1, wherein the starting material used is an adipic ester of the formula I comprising a total of from 0.01 to 4.5% by weight of hydroxycaproic ester and caprolactone.

3. A process as claimed in claim 1, wherein the starting material used is an adipic ester of the formula I comprising a total of from 0.1 to 3% by weight of hydroxycaproic ester and caprolactone.

4. A process as claimed in claim 1, wherein the reaction is carried out in the presence of water using a molar ratio of adipic ester to water within the range from 1:0.05 to 1:30.

5. A process as claimed in claim 1, wherein the catalyst used is selected from the group consisting of sodium oxide, potassium oxide or lanthanum oxide on the supports titanium dioxide, or zirconium dioxide and magnesium oxide or calcium oxide on zink oxide as support.

6. A process as claimed in claim 1, wherein the reaction is carried out in the presence of catalysts (a) and at from 220 to 350°C.

7. A process as claimed in claim 1, wherein the reaction is carried out in the presence of catalysts (b) and at from 300 to 450°C.

## Revendications

1. Procédé pour la préparation de la cyclopentanone par réaction d'esters adipiques de formule.
R₁OOC-(CH₂)₄-COOR₂ I
dans laquelle R₁ et R₂ représentent des groupes alkyle en C1-C12, cycloalkyle en C5 ou C6, aralkyle ou aryle, R₂ pouvant en outre représenter l'hydrogène, en présence de catalyseurs consistant en oxydes, **caractérisé par le fait que** l'on fait réagir des esters adipiques de formule I contenant moins de 5 % en poids de produits d'accompagnement autres que des esters adipiques, en phase gazeuse, en présence d'eau, d'un gaz véhicule et de
a) 0,01à 10 % en poids d'au moins un oxyde métallique choisi dans le premier ou le deuxième groupe principal de la Classification Périodique ou dans le groupe des métaux des terres rares sur dioxyde de titane ou dioxyde de zirconium servant de supports du catalyseur ou bien
b) 0,01 à 50 % en poids d'au moins un oxyde métallique choisi dans le deuxième groupe principal de la Classification Périodique, sur oxyde de zinc servant de support de catalyseur.

2. Procédé selon la revendication 1, **caractérisé par le fait que** l'on part d'esters adipiques de formule I contenant au total 0,1 à 4,5 % en poids d'esters hydroxycaproïques et caprolactone.

3. Procédé selon la revendication 1, **caractérisé par le fait que** l'on part d'esters adipiques de formule I contenant au total 0,1 à 3 % en poids d'esters hydroxycaproïques et caprolactone.

4. Procédé selon la revendication 1, **caractérisé par le fait que** la réaction est réalisée en présence d'eau, à un rapport molaire ester adipique/eau de 1 : 0.05 à 1 : 30.

5. Procédé selon la revendication 1, **caractérisé par le fait que** l'on utilise un catalyseur choisi dans le groupe consistant en l'oxyde de sodium, l'oxyde de potassium ou l'oxyde de lanthane sur support consistant en dioxyde de titane ou dioxyde de zirconium et oxyde de magnésium ou oxyde de calcium sur support consistant en oxyde de zinc.

6. Procédé selon la revendication 1, **caractérisé par le fait que** l'on réalise la réaction en présence des catalyseurs (a) à des températures de 220 à 350° C.

7. Procédé selon la revendication 1, **caractérisé par le fait que** l'on réalise la réaction en présence des catalyseurs (b) à des températures de 300 à 450° C.
